# EUROPÄISCHE PATENTSCHRIFT

(11) **EP 2 092 041 B1**
(45) Veröffentlichungstag und Bekanntmachung des Hinweises auf die Patenterteilung: **15.09.2010**
(21) Anmeldenummer: 07822496.1
(22) Anmeldetag: 12.11.2007
(51) Int. Cl.: C09K 11/06, H01L 51/54

(54) **NEUARTIGES HOCHLEITFÄHIGES ORGANISCHES LADUNGSTRÄGERTRANSPORTMATERIAL**
NOVEL HIGHLY CONDUCTIVE ORGANIC CHARGE CARRIER TRANSPORT MATERIAL
NOUVEAU MATÉRIAU DE TRANSPORT PORTEUR DE CHARGE ORGANIQUE ET HAUTEMENT CONDUCTEUR

(30) Priorität: 14.11.2006 DE 102006053644
(43) Veröffentlichungstag der Anmeldung: 26.08.2009
(73) Patentinhaber: Osram Opto Semiconductors Gmbh, 93055 Regensburg (DE)
(72) Erfinder: KANITZ, Andreas, 91315 Höchstadt (DE); ADLER, Jürgen, 91341 Röttenbach (DE); KRAUSE, Ralf, 91058 Erlangen (DE); SCHMID, Günter, 91334 Hemhofen (DE)
(74) Vertreter: Epping - Hermann - Fischer
(86) Internationale Anmeldenummer: PCT/EP2007/062210
(87) Internationale Veröffentlichungsnummer: WO 2008/058929

(56) Entgegenhaltungen:
- EP-A- 0 690 052
- R. GOMPPER ET. AL.: "Synthesis of Oligo(diazaphenyls). Tailor-Made Fluorescent Heteroaromatics and Pathways to Nanostructures" SYNTHESIS, Bd. 6, 1997, Seiten 696-708, XP002469293 Thieme

## Beschreibung

Die Erfindung betrifft ein organisches Material, insbesondere ein Material das als Halbleitermaterial oder leitfähiges Material in der organischen Elektronik eingesetzt werden kann.

Organische Halbleitermaterialien werden in Loch- und Elektronentransportmaterialien unterteilt. Diese werden zur Fertigung organischer elektronischer Bauelemente, wie beispielsweise Organische Leuchtdioden (OLEDs), Organische Feldeffekttransistoren (OFETs) und/oder Organische Solarzellen benötigt.

Auf der Seite der Lochtransportmaterialien wurden in den letzten 15 Jahren sehr effiziente und stabile Strukturen entwickelt, die je nach Anwendung mit unterschiedlichsten Lochinjektionseigenschaften zur Verfügung stehen und im lochtransportierenden, oxidierten Zustand stabile Radikalkationen bilden.

Auf der Seite der Elektronentransportmaterialien gibt es bisher nur sehr wenige Vertreter die den hohen Anforderungen, die an Elektronentransportmaterialien für die organische Elektronik genügen. Als gute Elektronenleiter gelten zurzeit die Derivate des Phenanthrolin (BCP und BPhen) sowie Derivate des Oxadiazols. Die radikalanionschen Spezies, die während des Betriebes dieser Bauelemente gebildet werden, führen in den heterocyclischen Strukturen eine Geometrieänderung herbei, so dass die Elektonentransporteigenschaft als Folge der Ausbildung von Konjugationsunterbrechungen abnimmt.

Defizite weisen die bekannten Materialien sowohl in der Bandbreite der Elektroneninjektion als auch in ihrer Stabilität im elektronentransportierenden, reduzierten Zustand auf, so dass insbesondere die Radikalanionen über einen längeren Zeitraum nicht reversibel gebildet werden können.

In der Regel werden die halbleitenden Eigenschaften der Materialien durch Dotierung erreicht, weil die Basismaterialien allein nie leitfähig genug sind.

Das Dokument EP 0 690 052 offenbart Elektrolumineszenzmaterialien, die aus konjugierten Verbindungen bestehen, die mindestens zwei oder mehr Pyrimidin-ringe enthalten. Diese konjugierten Verbindungen können entweder als lichtemittierende Materialien oder als Transportmaterialien in organischen lichtemittierenden Dioden eingesetzt werden

Aufgabe der vorliegenden Erfindung ist es deshalb, ein organisches Elektronentransportmaterial zu schaffen, bei dem die Bandbreite der Elektroneninjektion und auch die Stabilität im elektronentransportierenden, reduzierten Zustand gegenüber den bekannten Materialien verbessert ist. Insbesondere ist es Aufgabe der Erfindung, ein Material zu schaffen, das Radikalanionen über einen längeren Zeitraum reversibel bilden kann.

Zur Lösung der Aufgabe wurde ein effizienterer Elektronenleiter entwickelt, der sich durch eine größere Injektionsbandbreite und vor allem durch die Fähigkeit zur Bildung reversibler Radikalanionen hoher Stabilität auszeichnet.

Gegenstand der Erfindung ist ein Organisches Ladungsträgertransportmaterial, mit dem Elektronen und Löcher gleichermaßen gut transportiert werden können, wobei in einer 150nm dicken Schicht des undotierten Materials bereits bei 0,25V eine messbare Stromdichte von größer/gleich 0,5 A/cm² erhalten wird, und das Ladungsträgertransportmaterial Oligophenylene der heterocyclischen Stammverbindung der folgenden Struktur umfasst:

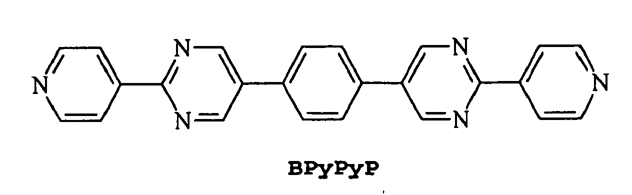

Ein BPyPyP-Material mit der oben gezeigten Struktur eines Oligophenylens, das im ursprünglichsten Sinn organisch ist, also nur Kohlenstoff, Stickstoff und Wasserstoff enthält, zeigt eine für rein organische Materialien (also nur C, H und N enthaltende Strukturen) ungewöhnliche Eigenschaft mit einer bisher um mehrere Größenordnungen unerreichten Leitfähigkeit bei der Elektronen und Löcher gleichermaßen transportiert werden.

Insbesondere werden bei einer 150nm dicken Schicht bereits bei 0,25V Stromdichten von 0,6A/cm² in positive und negative Richtung erreicht.

Nach einer besonders vorteilhaften Ausführungsform ist die Schicht dabei transparent, insbesondere bevorzugt im gesamten sichtbaren Spektralbereich transparent.

Die hier bei dem undotierten Material erreichte Stromdichte entspricht einer Stromdichte, die bisher nur durch so genannte dotierte Systeme erreicht wird.

Dabei ist neben den aufwendigen Prozessschritten zur Dotierung noch nachteilig an den dotierten Materialien, dass sie durch die Dotierung gewöhnlich einen Charge-Tranfer-Komplex bilden und im langwelligen Bereich des sichtbaren Spektrums absorbieren, also nicht transparent sind. Beide Nachteile werden erfindungsgemäß durch den Einsatz dieser einzigen Komponente, des BPyPyP im Material überwunden.

Daher kann dieses BPyPyP-Material direkt anstelle einer n-gedopten Elektonentransportmatrix zur Verbesserung der Injektion von Elektronen aus der Kathode in einem organischen elektronischen Bauelement eingesetzt werden.

Nach einer vorteilhaften Ausführungsform der Erfindung wird das BPyPyP Material durch Coverdampfen mit n-Dopingmaterialien oder N-Dotierungsmitteln als noch höher leitfähige Schicht abgeschieden.

Da das BPyPyP-Material ein hervorragender Elektonentransporter ist, lässt es sich in allen organischen elektronischen Bauelementen und Anordnungen in dieser Funktion einsetzten.

Darüber hinaus kann das Material ebenso in OLED-Anordnungen als ambipolare Matrix allein und in beliebigen Mischungen (blends) für beliebige Emittermaterialien eingesetzt werden.

Andererseits können aus dem Material und aus Mischungen mit diesem Material auch transparente Elektroden auf beliebigen Substraten erzeugt werden, die anodische und/oder kathodische Funktion haben.

Der hervorragende Stromtransport scheint durch eine selbstorganisierende geordnete Abscheidung der Moleküle durch den Aufdampfprozess zustande zu kommen.

Im Folgenden wird die Erfindung noch anhand von drei Figuren näher erläutert:

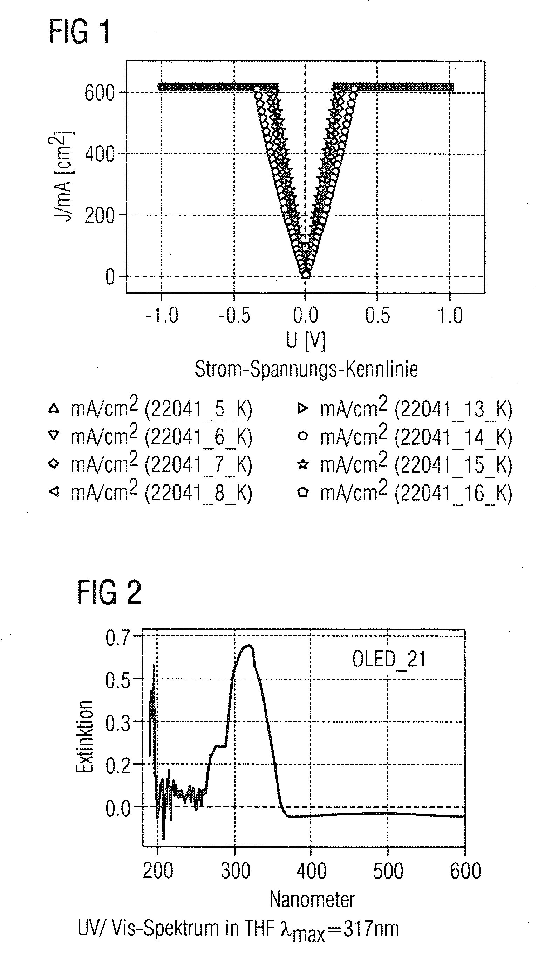

Figur 1 zeigt die Strom-Spannungs-Kennlinie, aus der hervorgeht, wie sich die Leitfähigkeit des Materials im positiven und negativen Spannungsfeld zeigt.
Figur 2 zeigt im UV/VIS-Spektrum die Transparenz des Materials und

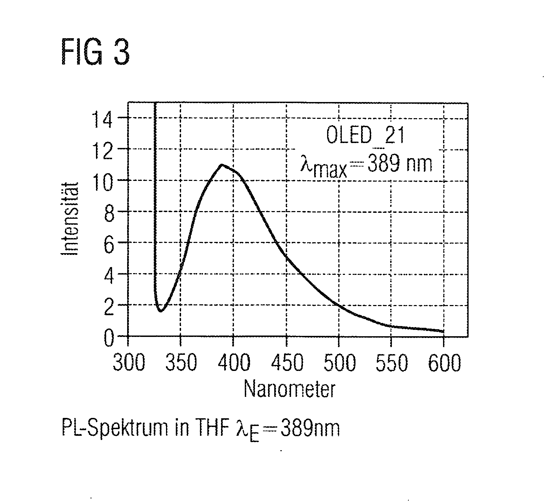

Figur 3 schließlich zeigt ein Photoluminsezenz-Spektrum der Verbindung.

Ein typischer Aufbau eines organischen elektronischen Bauelements umfasst ein Substrat, eine untere Elektrode, die halbleitende organische Schicht und eine obere Anode. Mit einem entsprechenden Aufbau wurden die in den Figuren enthaltenen Messdaten erhalten. Im gezeigten Beispiel war das Substrat aus Glas, die untere Elektrode aus Indium-Tin-Oxide (ITO), die halbleitende organische Schicht war eine 150 nm-dicke Schicht aus BPyPyP mit oben einer zweiten Elektrode aus einer 100 nm dicken Aluminium Schicht.

### Ausführungsbeispiele:

### Darstellung von Bis-2-(4-pyridyl)-pyrimid-5-yl-1,4-phenylen 1) 4-Pyridincarboxamidin Hydrochlorid 1

In einem 500 ml Kolben wird 4-Cyanopyridin (50,0 g, 470 mmol, 1 Äquiv.) zusammen mit Natriummethanolat (3,0 g, 53 mmol, 0,11 Äquiv.) in ca. 230 ml trockenem Methanol für 1,5 h bei RT gerührt. Zu der weißlichen Suspension wird Ammoniumchlorid (27,7 g, 52 mmol, 1,1 Äquiv.) gegeben und man lässt für mind. 24 h weiterrühren. Der schöne, fein verteilte, weiße Niederschlag wird abgesaugt und die Mutterlauge bis zur Trockene am Rotationsverdampfer eingeengt. Der Filterkuchen wird mit dem Rückstand aus der Mutterlauge vereinigt und aus Wasser umkristallisiert. Nach dem Absaugen und Waschen mit Ether wird das Produkt im Vakuum bei RT getrocknet.
Ausbeute: 70,5 g (95 % d.Th.), weiße Kristalle, M = 157,6 g·mol⁻¹; Schmelzpunkt: 250 °C

### 2) Bis(1,4- phenylen)-2-(3-dimethylamino-2-propen-dimethyliminium)-di-perchlorat 2

In einem 1000 ml Dreihalskolben mit Thermometer, Tropftrichter und Rückflusskühler mit aufgesetztem Trockenrohr wird DMF (200 ml, 2,5 mol, 30 Äquiv.) vorgelegt und mittels einer Eis/Salz-Kühlmischung auf ca. 0°C abgekühlt. Unter Rühren wird langsam Phosphoroxychlorid (46 ml, 0,5 mol, 6 Äquiv.) zugetropft, wobei die Temperatur nicht über 0°C steigen darf. Man lässt 10 min. nachrühren und gibt 1,4-Phenylendiessigsäure (16,3 g, 0,08 mol, 1 Äquiv.) zum Reaktionsgemisch. Anschließend wird die Lösung für 6 h auf 90°C erhitzt, abgekühlt und auf 1 kg Eis gegossen. Zur Fällung des Produktes wird Natriumperchlorat (30,8 g, 0,25 mol, 3 Äquiv.), gelöst in etwas Wasser, unter Rühren zur wässrigen Lösung gegeben. Der Niederschlag wird abgesaugt, mit Methanol/Ether und reinem Ether gewaschen und anschließend im Vakuum getrocknet.
Ausbeute: 31,5 g (71 % d.Th.), weiße Kristalle
M = 527,4 g·mol⁻¹; Schmelzpunkt: 292-296 °C

### 3) Bis-2-(4-pyridyl)-pyrimid-5-yl-1,4-phenylen

In einem 250 ml Kolben wird 4-Pyridincarboxamidin Hydrochlorid **1** (5,5 g, 35 mmol, 2,2 Äquiv.) zusammen mit dem Phenylenbisvinamidiniumsalz **2** (8,3 g, 18 mmol, 1 Äquiv.) in Pyridin für 8 h am RF erhitzt. Der entstandene braun-gelbe Niederschlag wird abgesaugt, mit Methanol, Methanol/Ether und reinem Ether gewaschen und im Vakuum bei RT getrocknet. Eine Umkristallisation kann aus DMSO erfolgen.
Danach wird das Produkt bei 320°C sublimiert.
Ausbeute: 4,5 g (74 % d.Th.), blassgelbe, nadelförmige Kristalle M = 388,4 g·mol⁻¹; Schmelzpunkt: >310°C;
DC: Kieselgel/THF, R_{F} = 0,81
Analytik: ¹H-NMR,

| | | |
|---|---|---|
| **¹H-NMR** **3** [D] TFA | 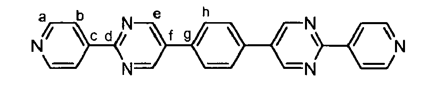 | a'+b') 9,42 (s, 8H) e') 9,98 (s, 4H) h') 8,44 (s, 4H) |

Die Erfindung betrifft ein organisches halbleitendes Material, insbesondere ein Material das als Halbleitermaterial oder leitendes Material in der organischen Elektronik eingesetzt werden kann. Dabei umfasst eine Komponente des Materials das Oligophenylen aus heterocyclischen Stammverbindungen der folgenden Struktur:

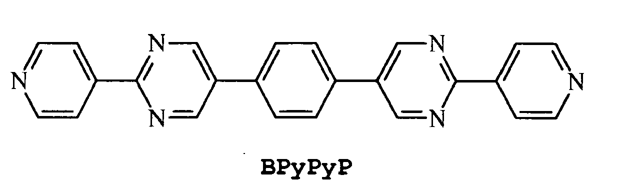

## Patentansprüche

1. Organisches Ladungsträgertransportmaterial, mit dem Elektronen und Löcher gleichermaßen gut transportiert werden können, wobei in einer 150nm dicken Schicht des undotierten Materials bereits bei 0,25V eine messbare Stromdichte von größer/gleich 0,5 A/cm² erhalten wird, und das Ladungsträgertransportmaterial Oligophenylene der heterocyclischen Stammverbindung der folgenden Struktur umfasst:

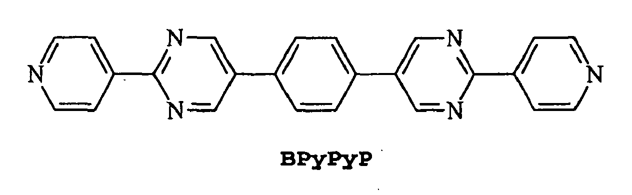

2. Ladungsträgertransportmaterial nach Anspruch 1, wobei das Material in dünnen Filmen transparent ist.

3. Ladungsträgertransportmaterial nach einem der vorstehenden Ansprüche, das als Mischung vorliegt.

4. Ladungstr.ägertrans.portmaterial nach einem der vors-tehenden Ansprüche, das n- oder p-dotiert vorliegt.

5. Verwendung eines Ladungstransportmaterials nach einem der vorstehenden Ansprüche in einem organischen elektronischen Bauelement.

6. Organisches elektronisches Bauelement mit einer halbleitenden Schicht und/öder leitfähigen Schicht auf der Basis eines Ladungsträgertransportmaterials nach einem der Ansprüche 1 bis 4.

## Claims

1. Organic charge carrier transport material with which electrons and holes can be transported equally well, wherein a measurable current density of greater than/equal to 0.5 A/cm² is already obtained in a 150 nm thick layer of the undoped material at 0.25 V, and the charge carrier transport material comprises oligophenylenes of the heterocyclic parent compound having the following structure:

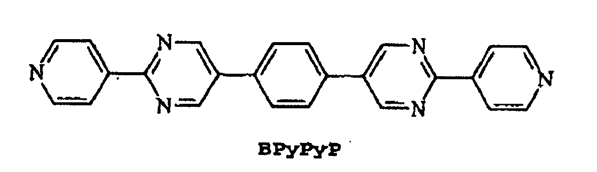

2. Charge carrier transport material according to Claim 1, wherein the material is transparent in thin films.

3. Charge carrier transport material according to either of the preceding claims, which is present as a mixture.

4. Charge carrier transport material according to any of the preceding claims, which is present in n- or p-doped fashion.

5. Use of a charge transport material according to any of the preceding claims in an organic electronic component.

6. Organic electronic component comprising a semiconducting layer and/or conductive layer on the basis of a charge carrier transport material according to any of Claims 1 to 4.

## Revendications

1. Matériau de transport porteur de charge organique, qui permet de bien transporter, de la même manière, des électrons et des trous, où on obtient, dans une couche d'une épaisseur de 150 nm du matériau non dopé déjà à 0,25 V une densité de courant mesurable supérieure/égale à 0,5 A/cm², et le matériau de transport porteur de charge comprend des oligophénylènes du composé souche hétérocyclique présentant la structure suivants :

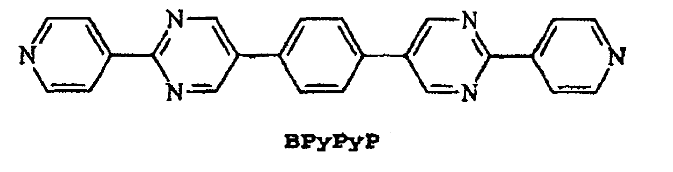

2. Matériau de transport porteur de charge selon la revendication 1, où le matériau en films minces est transparent.

3. Matériau de transport porteur de charge selon l'une quelconque des revendications précédentes, qui se trouve sous forme de mélange.

4. Matériau de transport porteur de charge selon l'une quelconque des revendications précédentes, qui se trouve sous forme n-dopée ou p-dopée.

5. Utilisation d'un matériau de transport de charge selon l'une quelconque des revendications précédentes, dans une pièce électronique organique.

6. Pièce électronique organique présentant une couche semi-conductrice et/ou une couche conductible à base d'un matériau de transport porteur de charge selon l'une quelconque des revendications 1 à 4.
